(19) Europäisches Patentamt
European Patent Office
Office européen des brevets

(11) **EP 2 994 488 B1**

(12) **EUROPEAN PATENT SPECIFICATION**

(45) Date of publication and mention
of the grant of the patent:
**13.07.2022 Bulletin 2022/28**

(21) Application number: **14724691.2**

(22) Date of filing: **08.05.2014**

(51) International Patent Classification (IPC):
**C07K 16/28** (2006.01)   **A61K 39/395** (2006.01)

(52) Cooperative Patent Classification (CPC):
**C07K 16/2896; A61P 19/02; A61P 29/00;
A61P 43/00;** A61K 2039/505; A61K 2039/54;
A61K 2039/545; C07K 2317/76

(86) International application number:
**PCT/EP2014/059477**

(87) International publication number:
**WO 2014/180961 (13.11.2014 Gazette 2014/46)**

(54) **USE OF C5AR ANTAGONISTS**

VERWENDUNG VON C5AR-ANTAGONISTEN

UTILISATION D'ANTAGONISTES C5AR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.05.2013 EP 13166973
09.05.2013 US 201361821336 P
21.01.2014 EP 14151982**

(43) Date of publication of application:
**16.03.2016 Bulletin 2016/11**

(73) Proprietor: **Novo Nordisk A/S
2880 Bagsværd (DK)**

(72) Inventors:
• **ANDERSSON, Ellen Christina
2880 Bagsværd (DK)**
• **NANSEN, Anneline
2880 Bagsværd (DK)**
• **USHER, Pernille Autzen
2880 Bagsværd (DK)**

(74) Representative: **Mewburn Ellis LLP
Aurora Building
Counterslip
Bristol BS1 6BX (GB)**

(56) References cited:
**WO-A1-2004/035079   WO-A1-2007/055374
WO-A1-2009/103113**

• **POBANZ J M ET AL: "C5A MODULATION OF
INTERLEUKIN-1BETA-INDUCED
INTERLEUKIN-6 PRODUCTION BY HUMAN
OSTEOBLAST-LIKE CELLS", JOURNAL OF
PERIODONTAL RESEARCH, BLACKWELL
MUNKSGAARD, COPENHAGEN, DK, vol. 35, no.
3, 1 June 2000 (2000-06-01), pages 137-145,
XP008052227, ISSN: 0022-3484, DOI:
10.1034/J.1600-0765.2000.035003137.X**
• **S. LIANG ET AL: "The C5a Receptor Impairs
IL-12-Dependent Clearance of Porphyromonas
gingivalis and Is Required for Induction of
Periodontal Bone Loss", THE JOURNAL OF
IMMUNOLOGY, vol. 186, no. 2, 15 January 2011
(2011-01-15), pages 869-877, XP055077599, ISSN:
0022-1767, DOI: 10.4049/jimmunol.1003252**
• **T. ABE ET AL: "Local Complement-Targeted
Intervention in Periodontitis: Proof-of-Concept
Using a C5a Receptor (CD88) Antagonist", THE
JOURNAL OF IMMUNOLOGY, vol. 189, no. 11, 22
October 2012 (2012-10-22), pages 5442-5448,
XP055077602, ISSN: 0022-1767, DOI:
10.4049/jimmunol.1202339**
• **T. BREIVIK ET AL: "Oral treatment with
complement factor C5a receptor (CD88)
antagonists inhibits experimental periodontitis in
rats", JOURNAL OF PERIODONTAL RESEARCH,
vol. 46, no. 6, 1 December 2011 (2011-12-01),
pages 643-647, XP055077604, ISSN: 0022-3484,
DOI: 10.1111/j.1600-0765.2011.01383.x**

- STEFAN RECKNAGEL ET AL: "C5aR-antagonist significantly reduces the deleterious effect of a blunt chest trauma on fracture healing", JOURNAL OF ORTHOPAEDIC RESEARCH, vol. 30, no. 4, 15 April 2012 (2012-04-15) , pages 581-586, XP055077612, ISSN: 0736-0266, DOI: 10.1002/jor.21561
- NELLI SHUSHAKOVA ET AL: "C5a anaphylatoxin is a major regulator of activating versus inhibitory Fc[gamma]Rs in immune complex-induced lung disease", JOURNAL OF CLINICAL INVESTIGATION, vol. 110, no. 12, 15 December 2002 (2002-12-15), pages 1823-1830, XP055077728, ISSN: 0021-9738, DOI: 10.1172/JCI200216577
- LEE H ET AL: "Human C5aR knock-in mice facilitate the production and assessment of anti-inflammatory monoclonal antibodies", NATURE BIOTECHNOLOGY, NATURE PUBLISHING GROUP, NEW YORK, NY, US, vol. 24, no. 10, 1 October 2006 (2006-10-01), pages 1279-1284, XP002481696, ISSN: 1087-0156, DOI: 10.1038/NBT1248 [retrieved on 2006-09-17] cited in the application
- LEE HYUN ET AL: "Receptors for complement C5a. The importance of C5aR and the enigmatic role of C5L2", IMMUNOLOGY AND CELL BIOLOGY, CARLTON, AU, vol. 86, no. 2, 1 January 2008 (2008-01-01), pages 153-160, XP009152114, ISSN: 0818-9641, DOI: 10.1038/SJ.ICB.7100166
- WHITFELD P ET AL: "Novel mAbs to C5aR 2nd loop reverse disease in models of inflammatory arthritis", IMFLAMMATION RESEARCH, BIRKHAEUSER VERLAG, BASEL, CH, vol. 56, no. Suppl.3, 1 June 2007 (2007-06-01), page S401, XP002547766, ISSN: 1023-3830
- ANDERSSON CHRISTINA ET AL: "Peripheral and Local Effects of Anti-C5aR Treatment in the Collagen Induced Arthritis Model", ARTHRITIS & RHEUMATISM, vol. 64, no. 10, Suppl. S, October 2012 (2012-10), page S881, XP002729042, & ANNUAL SCIENTIFIC MEETING OF THE AMERICAN-COLLEGE-OF-RHEUMATOLOGY (ACR) AND ASSOCIATION-OF-RHEUMATOL; WASHINGTON, DC, USA; NOVEMBER 09 -14, 2012
- A NANSEN ET AL: "Anti-c5a receptor antibody treatment ameliorates disease activity in delayed-type hypersensitivity (dth) arthritic mice -- Nansen et al. 72 (Suppl 3): A809 -- Annals of the Rheumatic Diseases", ANNALS OF THE RHEUMATIC DISEASES, vol. 72, no. Suppl. 3, 15 June 2013 (2013-06-15), pages 809-810, XP055137050, ISSN: 0003-4967
- C. ANDERSSON ET AL: "Rapid-onset clinical and mechanistic effects of anti-C5aR treatment in the mouse collagen-induced arthritis model", CLINICAL & EXPERIMENTAL IMMUNOLOGY, vol. 177, no. 1, 9 July 2014 (2014-07-09), pages 219-233, XP055137004, ISSN: 0009-9104, DOI: 10.1111/cei.12338

## Description

### TECHNICAL FIELD

The present invention concerns additional therapeutic uses of C5aR antagonists in particular in relation to bone damage and diseases and disorders related thereto.

### BACKGROUND

C5aR is the receptor for the A fragment of complement factor 5 (C5a). The receptor is expressed on a number of different cell types including leukocytes. C5aR belongs to the family of seven transmembrane G-protein-coupled receptors and is a high affinity receptor for C5a, with a Kd of ~1 nM. The structure of the receptor conforms to the seven transmembrane receptor family, with an extracellular N-terminus being followed by seven transmembrane helices connected by interhelical domains alternating as intracellular and extracellular loops, and ending with an intracellular C-terminal domain.

Inhibition of the C5a responses with C5aR antagonists reduces the acute inflammatory response mediated via C5a without affecting other complement components. To this end, C5aR peptide antagonists and anti-C5a receptor antibodies have been previously described (Watanabe et al., 1995 Journal of Immunological Methods 185: 19-29; Pellas et al., 1998; Konteatis et al., 1994; Kaneko et al., 1995; Morgan et al., 1993). For example, WO 95/00164 describes antibodies directed against an N-terminal peptide (residues 9-29) of C5aR. WO 03/062278 described antibodies reacting with the 2nd extracelluar loop (including clones referred to as 7F3, 6C12 and 12D4). Further C5aR antibodies have been described in WO/022390, WO 2009/103113 and recently WO2012/168199. All data confirming that effective blocking of C5a binding to its receptor C5aR, can inhibit C5a stimulated migration of neutrophils in vitro, and preventing inflammation in animal models.

C5a is a pro-inflammatory protein that has been implicated in inflammatory diseases such as in the pathogenesis of rheumatoid arthritis and the relevance of C5a and C5aR in rheumatoid arthritis has been confirmed in mice genetically deficient in C5aR, which are less prone to the induction of arthritis (Ji et al. (2002) Immunity 16: 157-168; Grant et al. (2002) J Exp Med 196: 1161-1171, Lee et al., 2006,(Nat Biotechnol. 2006 Oct;24(10):1279-84); Lee et al., 2008 Immunology and Cell Biology 86;153-160; Whitfeld et al., 2007 Inflammat. Res. 56(suppl. 3): S401; Andersson et al., 2012 Arthritis Rheumatism 2012, 64(10), Suppl. S: S881 and Hishimoto et al., 2010, J Exp Med. 2010 Jun 7;207(6):1135-43). Furthermore, treatment with a small molecule C5aR antagonist, a cyclic peptide can reduce inflammation and swelling after induction of arthritis in rats (Woodruff et al. (2002) Arthritis Rheum 46: 2476-2485).

Rheumatoid arthritis (RA) is an inflammatory disease of the joints. The affected joints are usually swollen and warm, and become increasingly more painful and stiff with time. Later symptoms include joint destruction including bone damage which severely affect the patient's movability and may include deformation of bones.

Although most drugs used for treatment of such inflammatory disease are mainly anti-inflammatory, it is of high importance and interest that a treatment of RA and related diseases also positively impact joint and bone destruction. In addition to RA, bone damage may occur in relation to other arthritis disease and even separate of an inflammation disease.

Osteoblasts and osteoclasts are collectively responsible for maintaining bones as osteoclasts may continuously break down osseous tissue while osteoblasts stimulate bone formation and thereby increase bone mass and/or bone density. Any compound that can influence the regulation of bone homeostasis and remodelling may thus be of relevance and have broad applicability in treatment of disease or disorders that affects the bones and in particular bone damage including bone erosion.

### SUMMARY

The inventors of the present application have found that C5aR antagonists have extended functionalities adding treatment of joint destruction, bone damage and in particular inhibition of bone erosion to the indications/symptoms where C5aR antagonists have usability.

The invention relates to a C5aR antagonist for use in a method for treatment or prevention of rheumatoid arthritis associated bone damage in a subject having rheumatoid arthritis, wherein the C5aR antagonist is an antibody that binds the 2nd extracellular loop of C5aR, wherein the antibody inhibits the binding of C5a to C5aR and inhibits the C5aR-mediated biological effect of C5a. Bone damage is here considered to include bone erosion and loss of bone density. In addition the C5aR antagonists may further be useful for the combined treatment or prevention of bone damage and cartilage degradation. Such treatment or prevention may be for reducing the rate of bone damage and/or for improving or increasing bone mineral density. Bone damage is at least partly a symptom of inflammatory diseases affecting the joints, in particular arthritis.

The inventors have surprisingly found that the effect of treatment using a C5aR antagonist provides a fast response as a therapeutic effect can be observed already after few dosages. In one embodiment the present invention relates to a C5aR antagonist for use in a method of treatment of prevention of bone damage, wherein an effect is obtainable after 4, 3, 2 or 1 dosage.

The functionality of the antagonists of the invention are considered linked to the ability of the antagonists to antagonize C5aR either by inhibiting or reducing binding of C5a to C5aR and by inhibiting or reducing C5aR mediated biological effect of C5a, such as a) C5a induced neutrophil activation, b) C5a induced cell migration and/or c) C5a induced neutrophil maturation.

A C5aR antagonist may be any type of compound suited for therapeutic administration including antibodies and peptide molecules. The C5aR antagonist according to the invention is an antibody. Disclosed herein are C5aR antagonists that would be administered as part of a pharmaceutical composition optionally including one or more pharmaceutical excipient.

## SEQUENCE LISTING

### SEQ ID NO.: 1: human C5aR

```
MDSFNYTTPD YGHYDDKDTL DLNTPVDKTS NTLRVPDILA LVIFAVVFLV GVLGNALVVW

VTAFEAKRTI NAIWFLNLAV ADFLSCLALP ILFTSIVQHH HWPFGGAACS ILPSLILLNM

YASILLLATI SADRFLLVFK PIWCQNFRGA GLAWIACAVA WGLALLLTIP SFLYRVVREE

YFPPKVLCGV DYSHDKRRER AVAIVRLVLG FLWPLLTLTI CYTFILLRTW SRRATRSTKT

LKVVVAVVAS FFIFWLPYQV TGIMMSFLEP SSPTFLLLKK LDSLCVSFAY INCCINPIIY

VVAGQGFQGR LRKSLPSLLR NVLTEESVVR ESKSFTRSTV DTMAQKTQAV
```

2nd loop is indicated by underlining of AA 171-206.

### SEQ ID NO.2: mouse C5aR

```
MNSSFEINYD HYGTMDPNIP ADGIHLPKRQ PGDVAALIIY SVVFLVGVPG NALVVWVTAF

EPDGPSNAIW FLNLAVADLL SCLAMPVLFT TVLNHNYWYF DATACIVLPS LILLNMYASI

LLLATISADR FLLVFKPIWC QKVRGTGLAW MACGVAWVLA LLLTIPSFVY REAYKDFYSE

HTVCGINYGG GSFPKEKAVA  ILRLMVGFVL PLLTLNICYT FLLLRTWSRK ATRSTKTLKV

VMAVVICFFI FWLPYQVTGV MIAWLPPSSP TLKRVEKLNS LCVSLAYINC CVNPIIYVMA

GQGFHGRLLR SLPSIIRNAL SEDSVGRDSK TFTPSTDDTS PRKSQAV
```

2nd loop is indicated by underlining of AA 167-203.

## BRIEF DESCRIPTION OF DRAWINGS

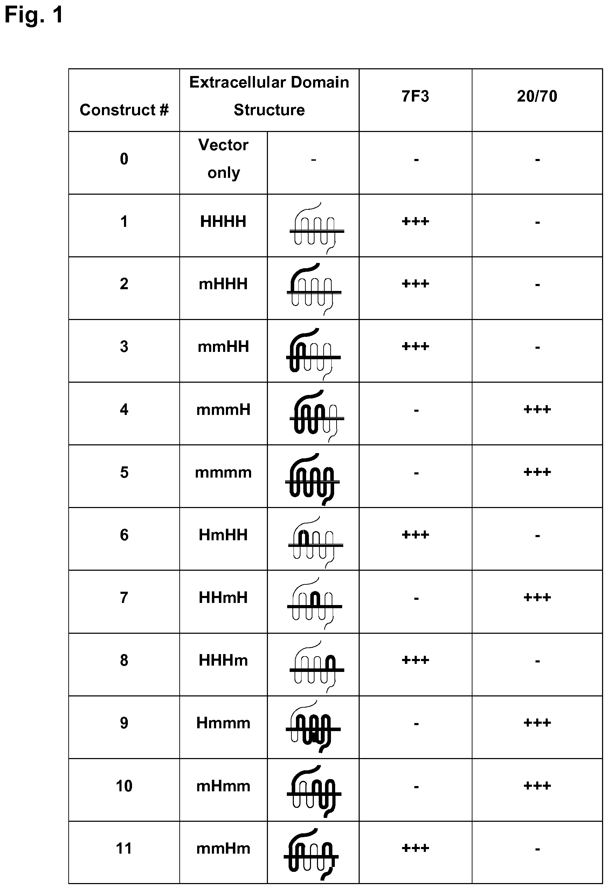

**Figure 1** shows the binding of anti-C5aR mAbs to chimeric human/mouse C5aR. The figure indicates which regions of the C5a receptors proteins that are derived from human and mouse, as the mouse sequences are shown with a bold line and the human sequences are depicted with a thin line.

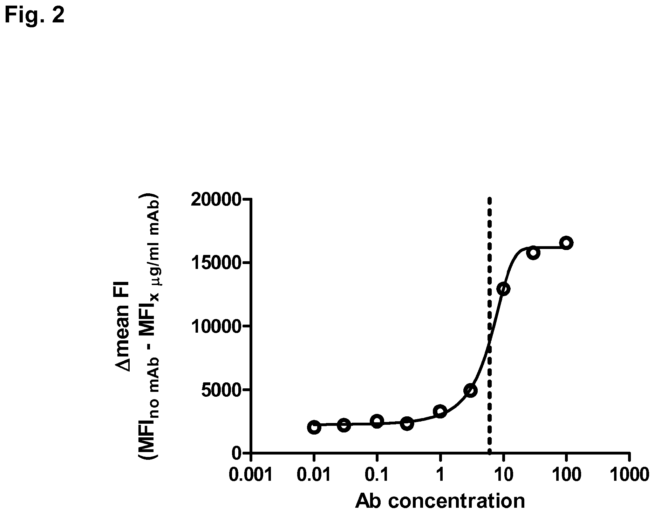

**Figure 2** shows C5a-induced CD11b expression quantified as $\Delta$ mean fluorescence intensity (FI): The mean FI value for a given anti-C5aR mAb concentration is subtracted from the mean FI value obtained from blood where no C5a is added. The result for mAb 20/70 is shown as a function of the mAb concentration. The dotted line represents the IC50 value, which was calculated to 0,6 $\mu$g/ml.

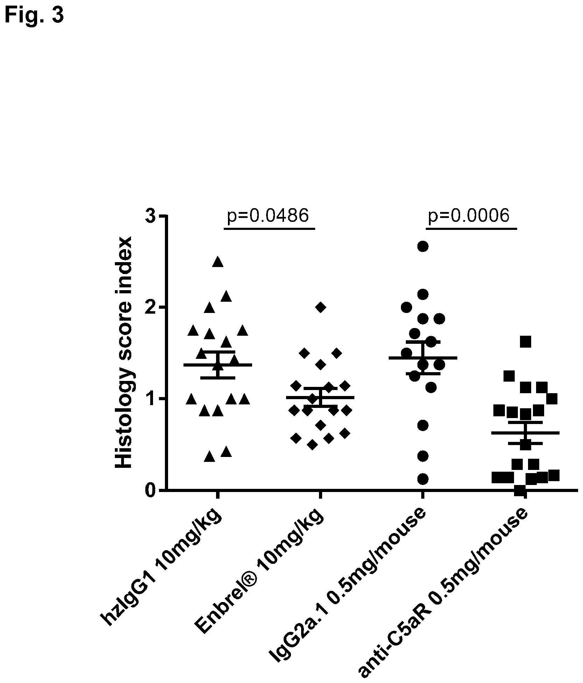

**Figure 3** shows that anti-C5aR reduces the histology score in the CIA mouse model. The graph shows the histology score index in mice treated with anti-C5aR, Enbrel® (Etanercept) and isotype control treatment, respectively. The histo-pathological end-point assessment of arthritic changes was performed on both hind paws from each mouse. Treatment with anti-C5aR significantly reduced the histology score index compared with the isotype control treatment.

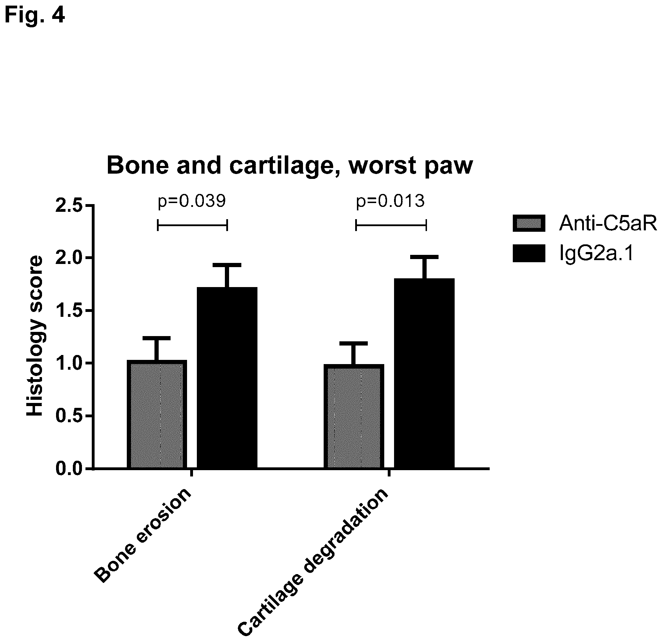

**Figure 4** shows that anti-C5aR inhibits bone erosion and cartilage degradation in the CIA mouse model. Bone erosion and cartilage degradation was evaluated on HE stained sections of worst affected hind paw from each mouse. Treatment with anti-C5aR significantly reduces both bone erosion and cartilage degradation compared with mice treated with anti-TNP control antibody.

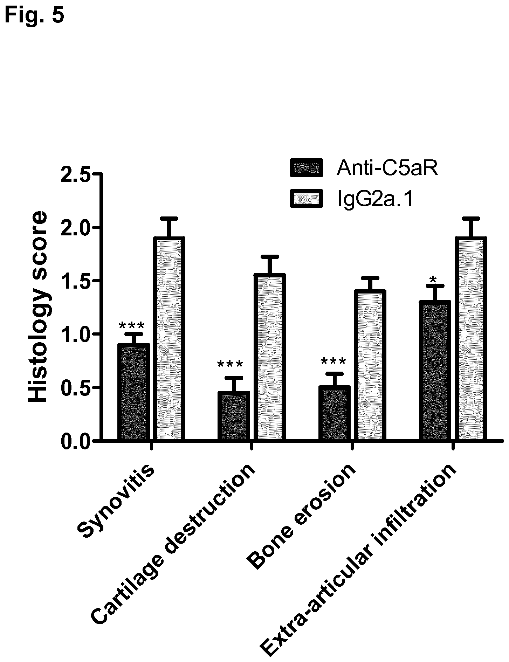

**Figure 5** shows that treatment with anti-C5aR reduces various arthritic parameters in a single paw arthritis model. Data was obtained from mice with DTH-arthritis treated with either blocking anti-C5aR antibody or isotype control antibody. Anti-C5aR treatment significantly reduces arthritic changes, including synovitis, cartilage degradation and bone erosion. In addition treatment significantly reduces extra-articular infiltration of inflammatory cells.

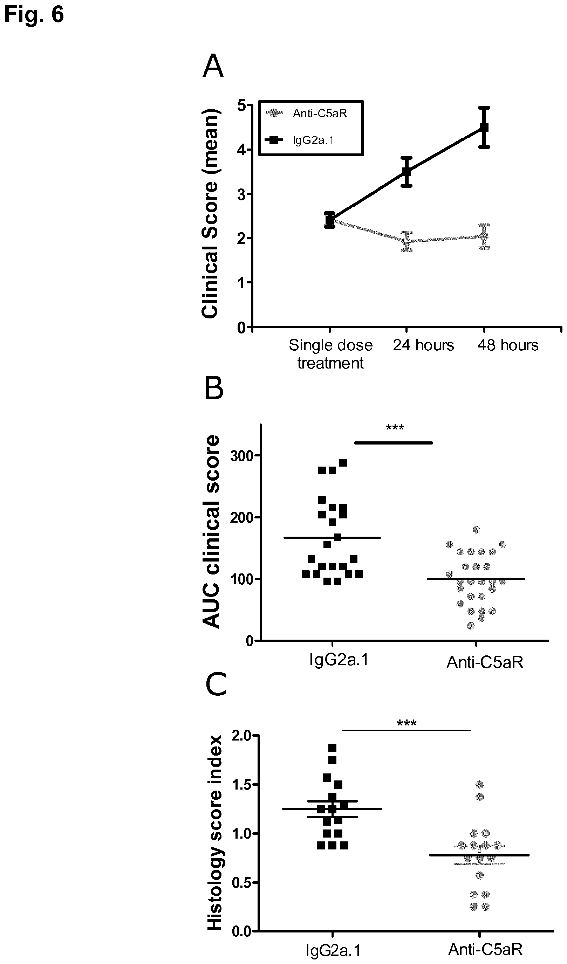

**Figure 6** shows that a single dose with anti-C5aR ameliorates arthritis 48 hours after treatment in the CIA mouse model. Figure 6A and 6B shows the mean clinical score ($\pm$SEM) per group over time and the AUC (area under the curve for individual clinical scores), respectively. The black line represents the mean. Figure 6C shows the Histology score index. Both the clinical and histopathological assessment shows significantly reduced disease.

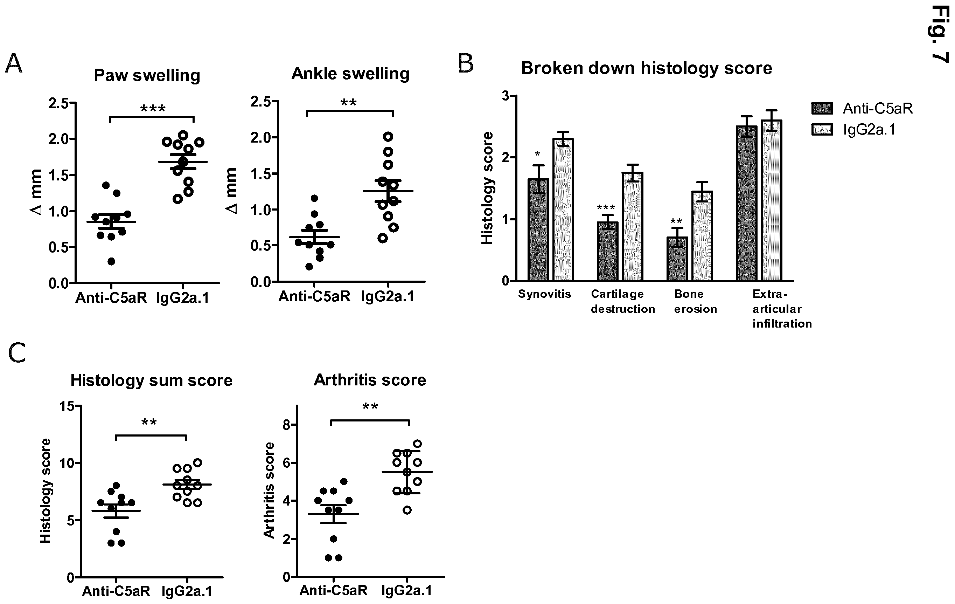

**Figure 7** shows that the disease activity in the DTHA model is reduced 60 hours following a single dose of anti-C5aR mAb. 7A shows the area under curve (AUC) of paw and ankle swelling in the two treatment groups measured over 60 hours after arthritis induction. Mean $\pm$ SEM shown, n=10. *: $p \leq 0.05$; **: $p \leq 0.01$, Student's t-test. 7B shows a Semi-quantitative histopathology scoring of arthritic and inflammatory parameters in the two treatment groups on a scale of 0-3. Mice treated with anti-C5aR displayed lower degrees of synovitis, cartilage destruction, and bone erosion. n=10. *: $p \leq 0.05$; **: $p \leq 0.01$; ***: $p \leq 0.001$, Student's t-test with Welch's correction. 7C shows the sum of the individual scores in B (arthritis sum score), and sum of the individual scores in B minus extra articular infiltration (arthritis score). Maximum possible score is 15 and 9, respectively. n=10.. *: $p \leq 0.05$; **: $p \leq 0.01$, Student's t-test with Welch's correction.

## DESCRIPTION

### Uses of C5aR antagonists

Compounds or drugs that inhibit or reduce a biological response usually elicited by ligand-receptor interaction are termed receptor antagonists. Such receptor antagonist will bind the receptor but the interaction will not have efficacy. Presence of an antagonist will thus inhibit or reduce the biological effect of the ligand (or ligands) of the receptor. The action of antagonists may be medicated by binding an active site of the receptor thereby blocking or disrupting ligand interaction. Alternatively an antagonist may bind the receptor at a different site which also effective prevents ligand binding or receptor signalling.

C5aR antagonists have been found to be of relevance in treatment of inflammatory diseases and disorders based on results from several disease models of particular arthritis. The signalling mediated by the C5a receptor upon binding of C5a may thus be inhibited or reduced resulting an inhibition or reduction of the on-going inflammation process and thus relief of the swelling of joints. In addition it has been found that C5a and C5aR have a function in relating to bone homeostasis and remodelling. The inventors of the present application have further established that C5aR antagonists have favourable effects on bone damage.

The present invention relates to a C5aR antagonist for use in a method for treatment or prevention of rheumatoid arthritis-associated bone damage in a subject having rheumatoid arthritis, wherein the C5aR antagonist is an antibody that binds the 2nd extracellular loop of C5aR, wherein the antibody inhibits the binding of C5a to C5aR and inhibits the C5aR-mediated biological effect of C5a. The invention is defined by the appended claims.

The ability of C5aR antagonists to affect the development of bone damage may relate to one or more of bone erosion and loss of bone density. In general, treatment is a very broad term and covers multiple aspects including any effect that is seen as an advantage for a patient that is suffering from or at risk of developing bone damage, such as one or more of bone erosion and loss of bone density. In the present description treatment is used when referring to situations where bone damage has been observed whereas prevention is used when referring to situations where bone damage has not yet been observed. It is noted that in the examples of this document "treatment" refers to the mere administration of a compound irrespectively of any therapeutic effect by this administration.

The C5aR antagonist may also be used in a method for treatment of bone damage (one or more of bone erosion and loss of bone density) and cartilage destruction.

Bone damage occurs by a continuous process that may result in severe effects on joint mobility. Bone damage in a given patient may have begun before that patient (or the physician) is aware that he or she is at risk of developing bone damage. In other cases the C5aR antagonist may be useful for prevention of bone damage in a patient at risk of developing bone damage. For example, the C5aR antagonist may be for use in a method for prophylactic treatment of bone damage.

The C5aR antagonist may also be used in a treatment to reverse or reduce bone damage e.g. to regenerate bone material of the joint. In a further embodiment the C5aR antagonist is for use in a treatment to improve bone mineral density.

It may be rare that bone damage can be fully prevented or fully treated and the ambition of a C5aR antagonist treatment may more often be to delay or halt progression of bone damage or to prevent further evolvement of bone damage.

In further embodiments the C5aR antagonist is for preventing bone damage, for reducing bone damage and/or for inhibiting bone damage. In further embodiments the C5aR antagonist if for delaying or halting of bone damage progression. In a further embodiment the C5aR antagonist is for inhibiting progression of bone damage.

In a further embodiment the C5aR antagonist is for use in a method for treatment of bone damage in a mammalian subject and in particular a human subject. As is apparent from the above the present invention relates to any aspect of treatment of bone damage in a human subject, such as preventing, inhibiting, halting and reversing of bone damage. Likewise the invention concerns treatment of bone damage including one or more of bone erosion and loss of bone density in a human subject.

The present invention thus relates to a C5aR antagonist for use in a method for treatment of bone damage in subject, wherein said subject is an individual in need. An individual in need is thus an individual that may benefit from treatment of bone damage, such as a patient suffering from bone damage or at risk of developing bone damage, again including one or more of bone erosion and loss of bone density.

The subject according to the invention has rheumatoid arthritis which may also include subjects that have Juvenile idiopathic arthritis (JIA), also known as juvenile rheumatoid arthritis (JRA).

The criteria for diagnosis of arthritis may differ over time and depend on local praxis and the time when a given patient is examined by a specialist capable of diagnosing arthritis and the different forms hereof. In one embodiment, the subject has one or more symptoms of rheumatoid arthritis.

In a further embodiment a C5aR antagonist is for use in a method for treatment of bone damage, such as inhibiting, halting and/or reversing of bone damage to obtain a fast response. The C5aR antagonist may be for use in a method for treatment of bone damage for obtaining a fast response. A fast response is considered present if it is detectable after a limited number of dosages, such as after at most 5 dosages, such as at most 3 dosages, or preferably after as few as two or even one dosage of the C5aR antagonist. Depending on the C5aR antagonist employed, different timing of dosing is expected and to be determined case by case. The time for considering if the C5aR may be used for obtaining a fast response may thus vary between. The fast response may also be detected or detectable before the 5th dosage is to be applied, such as before the 4th, 3rd or 2nd dosage is to be administered. The fast response may be detectable before a second dosage is to be administered. If a once weekly dosing is foreseen a response may thus be expected within a months from 1st dose, or within 4 weeks of 1st dose or within 3 weeks of first dose, or within 2 weeks of 1st dosage or within 1 week of first dose.

The fast response may be detected by various means known to the skilled artisan, as considered relevant for the disease and effect sought.

As the treatment of bone damage is not easily measurable in patients, alternative indirect means may be used.

The response may thus be detected by measurement of relevant disease biomarkers, such as markers describing the disease activity, such as cytokines and chemoattractants. Markers addressing the presence of specific cell types, such as MPO detecting neutrophils, as well as markers of tissue remodelling and/or extracellular matrix homeostasis may be used to evaluate if an early response is obtained. For clarity is noted that the fast response may be considered obtained also in situations where the effect is not actually measured e.g. situations where a response would have been detected if the relevant measurement had been performed.

A fast response may be detected (or detectable) by measuring relevant markers locally or in the periphery, where peripheral signals are preferably detectable in a serum sample.

The C5aR antagonist may be used in a method of treatment of bone damage or bone erosion seeking a fast response as describe above wherein a relevant change of a neutrophils marker, as cytokine marker, a chemoattractant markers or a tissue remodelling marker can be observed. Examples of suitable markers may be found in table 1 and obviously further markers may be available to the skilled person.

In a further embodiment, the C5aR antagonist is for oral administration. In a further embodiment, the C5aR antagonist is for intravenous administration. In a further embodiment, the C5aR antagonist is for subcutaneous administration.

In a further embodiment, the C5aR antagonist is for daily administration. In a further embodiment, the C5aR antagonist is for bi-weekly administration. In a further embodiment, the C5aR antagonist is for once weekly administration. In a further embodiment, the C5aR antagonist is for administration every second week. In a further embodiment, the C5aR antagonist is for administration with a frequency of every 20th -25th day, or such as once monthly.

**Methods of treatment**

An aspect of the invention relates to a method for treatment or prevention of bone damage according to the appended claims comprising administering a therapeutically effective amount of a C5aR antagonist to an individual in need.

**C5aR antagonists**

C5a, the A fragment of complement factor 5, binds its receptor C5aR and stimulates the inflammatory response. Inhibition of the C5a response with C5aR antagonists reduces the acute inflammatory response mediated via C5a without affecting other complement components. Different types of C5aR antagonists have previously been described (see background section) including peptide molecules such as cyclic peptide and anti-C5a receptor antibodies.

The critical feature for a C5aR antagonist is the ability of the molecule to interact specifically (in a given species) with C5aR, and not with other similar receptors such as C5L2, which is also a receptor for C5a. According to the invention a C5aR antagonist interacts specifically with C5aR. This interaction leading to inhibition or reduction of C5a binding to C5aR.

The terms "binding", "specifically binding" and "binding specificity" is use herein to describe the selectivity of a C5aR antagonist.

The functionality of an anti-C5aR antagonist is dependent on the ability of said antagonist to significantly inhibit or reduce binding of C5a to C5aR. In one embodiment the invention relates a C5aR antagonist capable of significantly inhibiting or reducing binding of C5a to C5aR. This may be determined by a displacement assay (SPA) as described in Example 2 herein, from which IC50 values can be determined. In one embodiment the IC50 is below 50nM. In a further embodiment of the invention the C5aR antagonist displaces C5a in an SPA assay, with an IC50 below 50nM, such as below 40nM, such as below 30nM, such as below 20nM, such as below 10 nM, such as below 5nM or even below 4nM, or with and IC50 below 3nM or even below 2.5nM or 2.0nM.

The term binding "affinity" is used to describe monovalent interactions (intrinsic activity). Binding affinity between two molecules, e.g. an antagonist and a receptor, through a monovalent interaction may be quantified by determination of the dissociation constant ($K_D$) by measurement of the kinetics of complex formation and dissociation, e.g. by surface plasmon resonance (SPR) method. The rate constants corresponding to the association and the dissociation of a monovalent complex are referred to as the association rate constant $k_a$ (or $k_{on}$) and dissociation rate constant $k_d$ (or $k_{off}$), respectively. $K_D$ is related to $k_a$ and $k_d$ through the equation $K_D = k_d / k_a$. Furthermore, "affinity" relates to the strength of the binding between a single binding site of a molecule (e.g., an antagonist) and a ligand (here receptor). The affinity of a molecule X for a ligand Y is represented by the dissociation constant ($K_d$), which is the concentration of Y that is required to occupy the combining sites of half the X molecules present in a solution. A smaller $K_d$ indicates a stronger or higher affinity interaction, and a lower concentration of ligand is needed to occupy the sites. Similarly, the specificity of an interaction may be assessed by determination and comparison of the $K_D$ value for the interaction of interest, such as a specific interaction between an antagonist and a receptor, with the $K_D$ value of an interaction not of interest.

The term "significantly" is used to describe that an effect is of biological relevance, such as at least 10 or 20 % inhibition or such as at least 10 or 20 % induction.

The affinity of a C5aR antagonist may alternatively be determined in a competition ligand binding assay performed using neutrophils. This functionality is referred to as affinity of the antagonist as measured in a competition assay, but could also be considered measurement of the avidity of the interaction. The *ex-vivo* assays measures the ability of C5aR antagonist to neutralize C5a mediated actions in an *in-vitro* setting. In one embodiment the C5aR antagonist has an affinity below 1.0 nM or 0.80 nM, such as below 0.50 nM or 0.35 nM, as measured by competition ligand binding assay on neutrophils.

A further functional characteristic of a C5aR antagonist is the ability to inhibit C5a-dependent migration of neutrophils. This functionality may be evaluated as described in Example 2 herein. Examples of antibodies displaying this functionality have been described in WO2012/168199. In one embodiment the invention thus relates to a C5aR antagonist, wherein said C5aR antagonist inhibits C5a induced cell migration *(in vitro* or *in vivo).*

In one embodiment the invention relates to a C5aR antagonist capable of significantly inhibiting migration of human neutrophils.

In one embodiment the C5aR antagonist significantly inhibits migration of neutrophils *in vitro.*

In one embodiment, the antibody inhibits migration to less than 50 %, less than 40 %, less than 30 %, less than 20 %, or less than 10 % compared to the level of migration observed in the presence of 10 nM C5a and no C5aR antagonist. In one such embodiment, the migration can be measured after 30 minutes in the presence of 10 nM C5a and C5aR antagonist and compared to the level of migration observed after 30 minutes in the presence of 10 nM C5a and no C5aR antagonist. Alternatively the ability of a C5aR antagonist to inhibit neutrophil migration can be express using IC50 values based on the same set up. In one such embodiment the IC50 is below 2.5 $\mu$g/ml, such as below 2.5 $\mu$g/ml, such as below 1.5 $\mu$g/ml, such as below 1.2 $\mu$g/ml or even below 1.0 $\mu$g/ml.

A further method to determine in vitro the functionality of a C5aR antagonist is a calcium-flux assay, that measures the ability of an C5aR antagonist to inhibit C5a induced neutrophil activation ex vivo, likewise described in Example 2.

In a further embodiment, the C5aR antagonist relates to an C5aR antagonist with an IC50 as determined in a calcium-flux assay below 7.0 $\mu$g/ml, such as below 5.0 $\mu$g/ml, such as below 2.5 $\mu$g/ml.

Additional ex vivo assays can be used to determine the ability of an C5aR antagonist to inhibit or neutralize C5a induced neutrophil maturation based on secondary effects such as CD11b and CD62L expression. CD11b and CD62L are maturation markers of neutrophils as they are up and down-regulated, respectively, upon activation by C5a/C5aR interaction.

In one embodiment, the C5aR antagonist relates to an C5aR antagonist with a IC50 as determined in an CD11b up-regulation assay is below 3.5 $\mu$g/ml, such as 3.0 $\mu$g/ml, such as below 2.5$\mu$g/ml, such as below 2.0$\mu$g/ml or such as 1.5 $\mu$g/ml or even below 1.0 $\mu$g/ml.

Likewise, the effect of the C5aR antagonist in a CD62L down-regulation assay may be determined. In one embodiment, C5aR antagonist relates to an C5aR antagonist with a IC50 as determined in a CD62L down-regulation assay is below 1.8 $\mu$g/ml, such as below 1.5 $\mu$g/ml, such as below 1.2 $\mu$g/ml or even below 1.0 $\mu$g/ml.

The skilled person will be aware of further criteria to determine if a given compound is a suitable C5aR antagonist and may thus choose an assay of preference.

Although C5a and C5aR molecules share similarity across species, a C5aR antagonist may be species specific, such as specific for the human complex or the mouse complex. In one embodiment, the antagonist is a human C5aR antagonist. In one embodiment, the antagonist is a mouse C5aR antagonist.

Different types of molecules may have C5aR antagonistic function. The C5aR antagonist of the invention is an antibody.

As used herein, the term "antibody" is used to describe whole antibodies and any antigen binding fragments (*i.e.,* "antigen-binding portion") or single chain molecules thereof which specifically bind its corresponding antigen, here C5aR. Examples of antigen-binding fragments include Fab, Fab', F(ab)2, F(ab')2, F(ab)S, Fv (typically the VL and VH domains of a single arm of an antibody), single-chain Fv (scFv), dsFv, Fd (typically the VH and CHI domain), and dAb (typically a VH domain) fragments; VH, VL, VhH, and V-NAR domains; monovalent molecules comprising a single VH and a single VL chain; minibodies, diabodies, triabodies, tetrabodies, and kappa bodies (see, e.g., III et al.. Protein Eng 1997;10:949-57); camel IgG; IgNAR; as well as one or more isolated CDRs or a functional paratope, where the isolated CDRs or antigen-binding residues or polypeptides can be associated or linked together so as to form a functional antibody fragment. Various types of antibody fragments have been described or reviewed in, e.g., Holliger and Hudson, Nat Biotechnol 2005;2S:1126-1136; WO2005040219, and published U.S. Patent Applications 20050238646 and 20020161201. Methods for generating antibodies, whole antibodies or antigen binding fragments are known in the art. Mouse monoclonal antibodies are typically made by fusing myeloma cells with the spleen cells from a mouse that has been immunized with the desired antigen. Human monoclonal antibodies can be obtained from transgenic animals (e.g. mice or other suitable species) encoding human antibodies. Alternatively, recombinant monoclonal antibodies can be made involving technologies, referred to as repertoire cloning or phage display/yeast display. Recombinant antibody engineering involves the use of viruses or yeast to create antibodies, rather than mice. Recombinant technologies may also be used for generation of "humanized" antibodies, which herein refers to a human/non-human chimeric antibody that contains sequences, usually at least the minimal complementarity-determining regions (CDR sequences) derived from a non-human germ line immunoglobulin sequence. A humanized antibody is, thus, a human immunoglobulin (recipient antibody) in which residues from a hyper-variable region of the recipient are replaced by residues from a hyper-variable region of a non-human species (donor antibody) such as from a mouse, rat, rabbit, or non-human primate, which have the desired specificity, affinity, and capacity.

The C5aR antagonist of the invention is an antibody binding the 2nd loop of C5aR. For example, the C5aR antagonist is an antibody binding the 2nd loop (AA 175-206) of human C5aR, an antibody binding AA 179-186 (EEYFPPKV) or the C5aR antagonist is an antibody binding the corresponding 2nd loop of mouse C5aR.

The antibody may be a monoclonal antibody, such as an antibody with the CDR sequences or variable regions of one of the antibodies described in any one of WO 03/062278, WO/022390 and WO2012/168199. A C5aR antagonistic antibody may be described as isolated to indicate that an antibody that has been separated and/or recovered from another/other component(s) of its natural environment and/or purified from a mixture of components in its natural environment.

In one embodiment, the C5aR antagonistic antibody may be of the IgG isotype, such as IgG1, IgG2 or IgG4.

Antibodies, via the Fc domain, interact with various Fc receptors and it therefore relevant to consider if such interaction is favourable or not as described in WO2012/168199, such as an antibody with one or more Fc mutations selected from E233P, L234A or V234A or F234L or F234V, L235E or L235A, G236R or G236A, G237A, S239D, S254W, N297Q, L328R, A330S, P331S and 332E. In one embodiment, the antibody Fc is an IgG1 including 234A, L235E, G237A, A330S and P331S mutations. The antibody may additionally include a D327Q mutation.

In one embodiment, the antibody does not significantly induce phagocytosis of neutrophils *in vitro,* meaning that the level of phagocytosis is not significantly above background as measured in the absence of an anti-C5aR antibody. In one embodiment the antibody does not give rise to any detectable induction of phagocytosis. The assay for evaluating the level of phagocytosis may be performed using human neutrophils as described in Example 4 of WO2012/168199.

In an alternative assays the ability of anti-hC5aR antibodies to mediate cell depletion e.g. to induce ADCC (antibody dependent cellular cytotoxicity) and CDC (complement dependent cytotoxicity) may be evaluated. The assays apply C5aR expressing cells as target cells and effector cells (monocyte-depleted PMBCs) or complement containing sera to elicit the response. The assay is described further in Example 4 of WO2012/168199.

For the purpose of this application antibodies and fragments hereof are considered non-peptide compounds, as the term is here use in relation to smaller molecules. In one embodiment the C5aR antagonist is a non-peptide compound.

C5aR antagonists disclosed herein may be peptide molecules, such as a peptide derived from C5a or C5aR. The C5aR antagonist may be a cyclic peptide, such 3D53 (AcPHe-Orn-Pro-DCha-Trp-Arg) cyclized through the side chain of Orn and the terminal carboxylate (Wong et all, IDrugs. 1999 Jul;2(7):686-93) which have been shown to inhibit binding of C5a to whole PMNs and to inhibit PMN degranulation as well (Finch et all, J Med Chem. 1999 Jun 3;42(11):1965-74). The compound 3D53 is also known as PMX53. In addition, a further similar compound is PMX205 (Woodruff TM: FASEB J, Vol. 20 (9), 1407-1417, 2006).

In addition to PMX53 and PMX205 described above further C5aR antagonists may include one or more of MP-435 (Mitsubishi Tanabe/J&J), CCX-168 (ChemoCentryx/GSK), MED17814 (MedImmune), ARC1905 (Ophtotech), NOX-D19 (Noxxon Pharma), ADC-1004 (Alligator Biosciences) and NGD 2000-1 (Neurogen).

The therapeutically effective amount of a C5aR antagonist must be determined for each compound and will depend on multiple causes, such as the potency, the bioavailability and *in vivo* half-life. The dosage to be administered will thus vary from compound to compound. In one embodiment where the C5aR antagonist is an antibody, the antibody may be administered in doses from 0.010 mg/kg up to 6 mg/kg.

**Pharmaceutical formulations**

Also disclosed herein are pharmaceutical compositions and/or formulations, comprising a pharmaceutically acceptable carrier and a C5aR antagonist according to the invention.

The C5aR antagonist according to the invention may be used in the preparation of a pharmaceutical composition. Such a pharmaceutical composition may be prepared based on general knowledge in the field such as in the Pharmacopeia or Remington.

The pharmaceutical composition may comprise an antibody as described herein in combination with a pharmaceutically acceptable carrier. The formulation may be in the form of a liquid formulation or a dry formulation that is reconstituted in water or an aqueous buffer composition prior to administration. The formulation may be in the form of an aqueous formulation. The formulation may be a dry formulation in the form of a tablet or capsule. The formulation may be sterilized.

A pharmaceutical composition of antibodies described herein may comprise a salt and/or buffer, such as the compositions described in WO2011/104381.

The pharmaceutical composition of an antibody described herein may be suitable for multiple uses, such as the compositions described in WO2011/147921.

The pharmaceutical compositions of the C5aR antagonist may be for intravenous administration. The pharmaceutical compositions of the C5aR antagonist may be for subcutaneous administration.

The pharmaceutical compositions of the C5aR antagonist may be for oral administration.

The pharmaceutical compositions of the C5aR antagonist may be for daily administration. The pharmaceutical compositions of the C5aR antagonist may be for bi-weekly administration. The pharmaceutical compositions of the C5aR antagonist may be for once weekly administration. The pharmaceutical compositions of the C5aR antagonist may be for administration every second week administration. The pharmaceutical compositions of the C5aR antagonist may be for administration with a frequency of every 20th -25th day, or such as once monthly.

The invention may further be described by the Embodiments described here below. The findings are also illustrated by the Examples presented herein.

**Examples**

**Example 1**

**Binding region of 20/70 (mouse anti-mC5aR) and 7F3 (mouse anti-hC5aR)**

The binding regions of 7F3 and mAb 20/70 to human and mouse C5aR, respectively, were determined and described (Lee et al. (2006) Nat Biotech 24 (10) 1279-1284). Briefly, chimeric human/mouse C5a receptors were constructed using overlap-extension PCR. Synthetic oligonucleotide primers were used to amplify regions of the human or mouse C5aR gene. The required human and mouse gene segments were joined to form a complete coding sequence for C5aR plus a C-terminal tag of 10 amino acids from bovine rhodopsin. Each DNA sequence was inserted into the mammalian cell expression vector pcDNA3.1(+) (Invitrogen) downstream of the constitutive CMV promoter. Expression vectors were transfected into mouse L1.2 cells using Lipofectamine 2000 (Invitrogen) according to manufacturer's instructions. Approximately 1.6 $\mu$g supercoiled plasmid DNA was added to $8 \times 10^5$ cells. Transfected L1.2 cells were grown in RPMI 1640 (Gibco) supplemented with 10% bovine calf serum (Hyclone).

One day after transfection ~$6 \times 10^4$ cells were centrifuged at 1,500 rpm for 5 min, washed once with PBS and re-suspended in 100 ul PBS containing 2% (wt/vol) BSA and 0.1% (wt/vol) sodium azide (staining buffer) and purified 20/70 antibody (5 ug/ml). After 30 min at 4°C, cells were washed twice with staining buffer and re-suspended in 50 $\mu$l FITC-conjugated donkey anti-rat IgG (Jackson Laboratories) diluted 1:200 in staining buffer. After incubating for 20 min at 4°C cells were washed twice with staining buffer and analysed on the FACSCalibur (Becton-Dickinson) to determine the level of surface expression.

A series of chimeric receptors comprising segments of mouse and human C5aR were constructed to identify the region of the C5a receptor that antibody 20/70 bound. Each receptor construct contained none, 1, 2, 3 or all 4 of the mouse C5aR extracellular domains with the missing domains being the human C5aR sequence. The origin of the 4 extracellular domains (N-terminus, 1st, 2nd & 3rd extracellular loops) in each construct is shown schematically in Figure 1, along with a summary of the FACS data.

Figure 1 suggests that the anti-C5aR mAb 20/70 binds to the 2nd extracellular loop of mouse C5aR and that 7F3 binds to the corresponding 2nd loop in human C5aR. 20/70 binds to constructs 4, 5, 7, 9 and 10, all of which contain the mouse C5aR 2nd extracellular loop. Indeed, construct #7 comprises all human C5aR except for the 2nd loop which is mouse C5aR. Furthermore, 20/70 does not cross-react with human C5aR as evidenced by lack of binding to construct #1. Transfectants incubated in the presence of the FITC-conjugated secondary antibody only (i.e. no primary antibody) showed no staining (data not shown).

**Example 2**

*In vitro* **tests of C5aR antagonists**

CD11b receptor upreaulation

Capacity of anti-C5aR mAb (m20/70) to inhibit up-regulation of CD11b expression in response to C5a.

**Assay set-up**

The following set up was designed to determine the ability of C5aR antagonists to neutralize C5a-induced neutrophil maturation by measuring changes in CD11b expression.

**Materials and methods:**

Blood was collected into EDTA-coated tubes from naive C57BL/6 mice (Taconic, Denmark) by puncture of the submandibular plexus. After collection the blood was pooled and 10 $\mu$l of anti-C5aR mAb (m20/70, Shushakova et al. 2002, Hycult biotech) in different concentrations (see table) was added to 11 aliquots of 90 $\mu$l pooled blood and incubated for 20 min. at room temperature (RT). After incubation, 9 $\mu$l 10 $\mu$g/ml recombinant mouse C5a (Hycult Biotech, HC1101) in PBS with 0.1% BSA (Miltenyi Biotech, 130-091-376) was added to each tube, except tube 1, and incubated for 20 min. at RT. Then each sample was stained for flow cytometry using 50 $\mu$l of a mastermix containing saturating concentrations of anti-Ly6G-PE (BD Biosciences, 55161) and anti-CD11b-AF700 (eBiosciences, 56-0112-82) in a staining buffer containing Cellwash (BD Biosciences, 349524) and 0.2% BSA (Miltenyi Biotech, 130-091-376). For staining of dead cells Fixable Near IR dead cell stain kit (Invitrogen, L10119) was added to the staining cocktail according to the manufacturer's instructions. Following incubation for 20 min. at 4°C erythrocytes are lysed by adding FACS lysing solution (BD Biosciences, 349202) and incubating for 15 min. in the dark at RT. Cells are washed twice using staining buffer and subsequently analysed on a LSRII flow cytometer (BD Biosciences) using FACS Diva software.

| Tube | 1 | 2 | 3 | 4 | 5 | 6 | 7 | 8 | 9 | 10 | 11 |
|---|---|---|---|---|---|---|---|---|---|---|---|
| C5a (9 μl 10 μg/ml) | - | + | + | + | + | + | + | + | + | + | + |
| mAb μg/ml | 0.00 | 0.00 | 0.01 | 0.03 | 0.10 | 0.30 | 1.00 | 3.00 | 10 | 30 | 100 |

**Results:**

C5a-induced CD11b expression on neutrophils (defined as live Ly6G[+] cells) was quantified as mean fluorescence intensity (FI) of AF700 for a given anti-C5aR mAb concentration subtracted from the value obtained from blood where no C5a was added (Fig. 2). The IC50 value for the anti-C5aR mAb was calculated as 0,6 μg/ml using non-linear curve fitting in GraphPad Prism software (v. 5.03).

It follows that the assay may be adapted to test other compounds evaluating there relevance as C5aR antagonists.

*CD62L receptor down-regulation*

Assay set-up

The following set up is designed to determine the ability of C5aR antagonists to neutralize C5a-induced neutrophil activation by measuring changes in CD62L expression.

The above *CD11b* assay is adapted for CD62L detection by using a conjugated antibody recognizing CD62L (BD Biosciences, Cat. No 559772). The experimental details specific for CD62L are given below.

FACS and data analysis

The FACSCalibur flow cytometer (BD Biosciences) is setup with compensation parameters established for channel FL-4. Samples are gated to exclude dead cells and debris. Neutrophils are identified as having high FSC and SSC and gated. The mean fluorescence intensity (MFI) of the gated neutrophils in the FL-4 (CD62L-APC) channel is calculated.

Results are expressed as a percentage of maximum CD62L expression with background subtracted. Maximum CD62L expression (MaxCD62L) is the average MFI of the neutrophils incubated without C5a and without C5aR antagonist. The minimum (background) CD62L expression (MinCD62L) is the average MFI of the neutrophils incubated with C5a but without C5aR antagonist. The formula used to calculate % of maximum CD62L expression for each samples was:

$$\% \text{ Max}_{sample} = (MFI_{sample} - MFI_{Min}) / (MFI_{Max} - MFI_{Min}) \times 100$$

Data maybe entered into GraphPad Prism (v4.0) and fitted to the sigmoidal dose-response curve (variable slope) i.e. 4-parameter logistic equation using non-linear regression to calculate the EC50.

Displacement Assay

A Scintillation Proximity Assay (SPA) can be applied in order to determine the potency of an C5aR antagonist to displace C5a binding to C5aR. A detailed description of the SPA is provided in US patent 4568649 and protocols provided by the manufacturer (Amersham Biosciences). Briefly, receptor-carrying membrane fragments purified from RBL-hC5aR cells bind to scintillating micro particles coated with wheat germ agglutinin (WGA). After addition of radio-labelled hC5a ($^{125}$I) tracer, binding to the receptors will result in emission of light from the particles. Specific for the SPA-principle, only radio isotope and particles in immediate proximity of each other will emit light. I.e. only radio-labelled hC5a bound to a receptor is close enough to a WGA-particle to produce light. The amount of light emitted is thus an expression of the amount of receptor-bound $^{125}$I-hC5a. The assay is a competition assay, in which anti-hC5aR/unlabelled hC5a competes with the tracer on binding to the receptors. In the assay, a fixed amount of $^{125}$I-labelled C5a is added to WGA-particles and C5aR receptors resulting in emission of a certain amount of light measured as counts per minute (cpm). If unlabelled C5a or anti-C5aR is added, binding hereof to the receptors will cause a lower cpm due to displacement of $^{125}$I C5a. The % displacement is calculated as follows:

$$\frac{S - S\max}{So - S\max} \cdot 100\%$$

**S**: Sample

**S$_{max}$**: Non specific binding. Measured by adding unlabelled hC5a in an amount sufficient to supersede the specifically bound $^{125}$I-hC5a.

**S$_0$**: Maximum binding. No unlabelled hC5a is added.

The IC50 value is defined as the concentration which displaces 50% of C5a. The cpm is kept constant between experiments hence the IC50 values are relative as the tracer decades over time. The potency (IC50) of compounds to displace $^{125}$I-hC5a may be used to identify C5aR antagonist.

Neutrophil Migration (Chemotaxis) Assay

The potency of C5aR antagonists to inhibit hC5a (or mC5a) dependent neutrophil migration can be analysed in a Boyden chamber. Neutrophils isolated from human or animal blood are stained with calcein and added to the upper compartment in the Boyden chamber and mixed with the antibodies. hC5a or mC5a is applied to the lower compartment in the Boyden chamber and acting as chemoattractant for the neutrophils. The ability of neutrophils to migrate to the lower chamber is determined by counting the number of calcein-stained neutrophils passing through a 3 or 5$\mu$m fluoroblok membrane.

Human PMNs (PolyMorphoNuclear leukocytes; granulocytes) are obtained from human blood samples drawn into vials containing EDTA. The blood cells areseparated by centrifugation of blood (4 parts) through a Ficoll-Paque PLUS (GE Health Care) gradient (3 parts) for 30 min (400 $\times$ g) at room temperature. The PMN-containing layer is suspended in PBS (phosphate buffered saline) containing dextran-500 (Sigma) for 1 h to remove contaminating erythrocytes. The supernatant is centrifuged for 5 min (250 $\times$ g) at room temperature and remaining erythrocytes are osmotically lysed using 0.2% NaCl for 55 s. The solution is made isotonic by 1.2 % NaCl + PBS and centrifuged at 250 $\times$ g for 5 min, before the osmotic lysis is repeated. After centrifugation the PMNs are resuspended in reaction mixture (RM): HBSS (cat no 14175 Gibco) contains NaCl 137mM, KCl 5.3mM, Na$_2$HPO$_4$ 0.33mM, NaHCO$_3$ 4mM, KH$_2$PO$_4$ 0.44mM, Glucose 5mM; supplemented with MgSO$_4$•7H$_2$O 0.4mM, MgCl$_2$, 0.5mM, CaCl$_2$ 0.5mM, HEPES 20mM. Cell density is determined by NucleoCounter (Chemometec). The PMN suspension should contain >95 % neutrophils as evaluated by microscopy of Giemsa-stained samples.

Loading PMNs: Calcein, AM, (Fluka) is dissolved in DMSO (Dimethyl sulphoxide) and diluted 1000X in RM with cells (2$\times$10$^6$ cells per ml) to yield a concentration of 10 $\mu$M. The suspension is incubated for 30 min in incubator at 37°C and then washed 3 times with RM to remove excess Calcein. Finally the cells are resuspended in RM (4$\times$10$^6$ cells/ml),

Migration is evaluated by the Boyden chamber technique using FluoroBlok® 3$\mu$m pore size 96- well (cat. No. 351161.BD Falcon (VWR)). The upper chamber i.e. the inserts containing Fluoroblok membrane, is coated with human fibrinogen (cat no F3879-1G, Sigma) in 1 mg/ml PBS at 37°C for 2 hrs. After washing the membranes are blocked with a solution containing 2% bovine serum albumin (BSA), in PBS. After another wash using RM, 10$^5$ Calcein-loaded PMNs with or without the C5aR antagonist are added to each well and placed in the receiver plate (lower chamber) which contained the control solution or the chemoattractant hC5a (Sigma, C5788). Each group comprised of at least 6 wells. Thereafter the plate is measured at 485/538nm, 37°C every 5 min for 60 min in a plate reader (SpectraMax, Molecular devices, or Fluoroscan, Thermo Labsystems.). The value at 30 min in relative fluorescence units is used as a measure of migration.

Curve fitting. The ability of C5aR antagonists to inhibit migration may be expressed by IC50 as determined using GraphPad Prism 5 (GraphPad Software, Inc.)

*Ex-vivo* measurement of affinity

The assay measures the ability of C5aR antagonist to neutralize C5a mediated actions in an *in-vitro* setting.

Isolation of neutrophils from fresh human blood

Blood is diluted in 1:1 with PBS + 2% FBS and layered on Ficoll-Paque PLUS (GE Healthcare #17-1440-03) at a ratio of 3 parts Ficoll and 4 parts blood (15 ml Ficoll and 20ml blood in a 50 ml tube) and subsequently stratified by centrifugation at 400 $\times$ g for 30 minutes at RT. By aspiration the intermediate PBMC band is gently removed. The granulocytes stratified on the packed red cells are aspirated with a plastic Pasteur pipette. The granulocytes and red cells are transferred and pelleted in a new 50 ml tube. The pellet is diluted to 40 ml with 1x PBS and 10 ml of a 4% DEXTRAN 500 (sigma, 31392) solution in PBS (rate1:5) is added and mixed gently by inversion. After 20-30 min. the granulocyte rich supernatant obtained is transferred to a new tube and spun down at 250 $\times$ g for 5 min at RT. The contaminating red cells are removed with osmotic lysis by resuspending the cell pellet in 7.5 ml of 0.2 % NaCl and gently mixing for 55-60 seconds. Subsequently 17.5 ml of 1.2 % NaCl is added and then diluted to 50 ml with PBS and spun down at 250 $\times$ g for 5 min. This step is repeated once. The cell pellets are subsequently resuspended in 1 ml reaction mixture (dPBS/RPMI). The viability and cell count is monitored using NucleoCounter®.

Competition liaand binding assay on neutrophils

Human neutrophils are purified, washed and resuspended in binding buffer (50 mM HEPES, pH 7.5, 1 mM CaCl$_2$, 5 mM MgCl$_2$ and 0.5% bovine serum albumin (FractionV Ig G free)) at ~5 $\times$ 10$^6$ cells/ml. For each sample 40 $\mu$l cell suspension (1 $\times$ 10$^5$ cells/well) is seeded into a 96-well V-shaped plate (Greiner, Cat.# 651101). Competition studies are done using 12 concentrations of competing unlabeled ligand in half-log dilutions starting with 1 $\mu$M as the highest concentration. 40 $\mu$l of C5aR antagonist is added considering a final assay volume of 120 $\mu$l. 40 $\mu$l radioligand [$^{125}$I]-hC5a (Perkin Elmer, Cat. No. NEX250) is added to all samples except the background control. The final concentration of radioligand in the assay is 1 nM and the final volume is 120 $\mu$L. All samples are run in triplicate and incubated for 4h at 4°C. Cells are then collected by centrifugation at 1200 rpm, at 4°C for 2 min and washed three times in 100 $\mu$l of wash buffer (50 mM HEPES, pH 7.5, 1 mM CaCl$_2$, 5 mM MgCl$_2$, 150 mM NaCl and 0.5% bovine serum albumin (FractionV Ig G free)). Finally, cells are re-suspended in 30 $\mu$l wash buffer and transfered to an OptiPlate (Perkin Elmer, Cat. No. 6005290) and 150 $\mu$l of MicroScint 20 (Perkin Elmer, Cat. No. 6013621) is added to each well. The plates are covered, mixed well counted on a calibrated Top Counter with 1h delay. The total amount of radioligand added to the assay is determined on a separate plate. The number of counts in each sample is expressed as normalized values in percentage where 100% is the maximum level of counts when 1 nM [$^{125}$I]-hC5a and no cold C5aR antagonist is added, and 0 % is unspecific binding determined in the presence of 1 $\mu$M cold C5a. The data are analyzed by nonlinear regression using PRISM (GraphPad).

Calcium-flux assay

Staining of human neutrophils with Fluo-4 AM cell dye

Neutrophils are centrifuged and washed in PBS then resuspended at 1 $\times$ 10$^7$ cells/ml in Cell Dye and incubated at room temperature for 40 min in darkness. Cells are centrifuged and washed (to remove excess dye), centrifuged again and resuspended at 2 $\times$ 10$^6$ cell/ml in Cell Buffer. Cells (0.5 ml) are aliquoted into non-sterile glass FACS tubes - one tube for each sample - stored at room temperature and used within two hours. Each sample used 1 $\times$ 10$^6$ neutrophils.

Assay

The calcium flux assay is carried out as follows. Briefly, 1 $\times$ 10$^6$ neutrophils loaded with Fluo-4 AM in 0.5 ml Cell Buffer are analysed on a FACSCalibur flow cytometer (BD Biosciences) with neutrophils gated using x-axis FSC vs. y-axis SSC. The FL-1 (FITC) channel is used to measure neutrophil fluorescence following addition of various reagents to the tube (e.g. C5aR antagonist, C5a, ionomycin - dissolved at 10x final concentration in Cell Buffer rather than I-MGB or C-MGB). Sample fluorescence is measured continuously with a mean fluorescence intensity (MFI) value acquired every 1 second. This data is saved in a CellQuest (BD Biosciences) file and transferred to Excel (Microsoft) and Prism (v4.0c, GraphPad Software Inc.) for further processing and analysis. The order of adding reagents to the neutrophils and incubation times may be varied according to the type of assay carried out.

C5a neutralization assay

A 10x 3-fold serial dilution of C5aR antagonist, with concentrations ranging from 1000 $\mu$g/ml to 1,37 $\mu$g/ml, is prepared. Fluo-4 AM loaded neutrophils (1 $\times$ 10$^6$ in 0.5 ml Cell Buffer) are incubated with 50 $\mu$l 10x C5aR antagonist solution (final C5aR antagonist concentration in tube: 100 - 0.137 $\mu$g/ml) for 10 min at room temperature. Cells plus C5aR antagonist are analysed by FACS for -60 sec to establish baseline fluorescence. Then 50 $\mu$l 10 nM C5a is added to give a final concentration of ~1 nM and fluorescence measurement continued for another -60 sec. If a C5aR antagonist blocks C5a-induced Ca$^{2+}$-release then there is no spike of fluorescence. If the C5aR antagonist does not neutralize the C5a then there is a spike in fluorescence. Lastly, 50 $\mu$l 1 $\mu$g/ml ionomycin is added to a final concentration of 0,1 $\mu$g/ml and fluorescence measurement continued for another -60 sec to ensure cells are still responsive.

**Example 3**

**Effects of anti-C5aR in the collagen induced arthritis (CIA) model**

**Mice**

Male DBA/1 mice (10-11 weeks old) were obtained from Taconic (Denmark) and were allowed to rest for one week before initiation of the experiment. The mice were housed (10 mice/cage) under standard conditions and received standard diet and water.

Immunization for induction of CIA

Mice were immunized intradermally (i.d.) at the base of the tail with 100 $\mu$l bovine type II collagen (bCII, 2 mg/ml, Chondrex, USA) emulsified 1:1 in complete freunds adjuvans (2 mg/ml, Arthrogen-CIA®Adjuvant, Chondrex, USA) on day 0. On day 21 mice were given a boost immunization i.d. at the base of the tail with 100 $\mu$l bCII (1 mg/ml, Chondrex, USA) emulisified 1:1 with incomplete freunds adjuvans (Chondrex, USA). Mice were immunized under anaesthesia using isoflourane/$O_2$/$N_2O$.

**Clinical scoring of arthritis**

Mice were scored from day 21 to the end of the experiment 5 times per week (all days except weekend days) according to the following scoring key: 0 points if no visible clinical symptoms

1 point/swollen toe (regardless of whether this includes one or two joints (- digit I in front paws), 1 point for involvement of knuckles, metatarsal/metacarpal, 1 point for involvement of wrist, tarsal/carpal. A maximum score of 6 could be obtained in front paws and a maximum score of 7 could be obtained in the hind paws. Mice that obtained a clinical score of more than 10 before experiment termination were sacrificed according to the definition of humane endpoints by the local Danish authorities.

**Anti-mouse C5aR mAb (m20/70)**

The variable region for HC and LC was derived from the rat anti-mouse anti-C5aR mAb 20/70 (Shushakova et al. 2002, Hycult biotech).The constant region of LC is mouse kappa and constant region of HC is mouse IgG2a designed with 6 mutations in the FC region resulting in mIgG2a.1. The engineered mutations L234A, L235E, G237A (ADCC inactivation), D327Q, A330S and P331S (CDC inactivation) were made to reduce ADCC or CDC effector mechanisms. Lack of binding to mouse Fc-gamma receptors I-IV was determined by surface plasmon resonance analysis (data not shown).

**Test compounds and treatment of mice**

Individual therapeutic treatment of mice was initiated when mice obtained a score of 2-7 (most mice had score 2-4 at treatment initiation). Mice were randomized according to the treatment so that the first mouse qualifying for treatment initiation were given compound 1 and second mouse qualifying for treatment initiation were given compound 2 and so on. Using this procedure all compounds were represented in all cages and both early and late arthritis onset mice were represented in all dosing groups. All mice received either 6 doses over two weeks (with 1-2 days between treatments because no treatments were given during weekends). Mice were treated intraperitoneally (i.p.) with one of the following compounds:

20/70 anti-C5aR (mouse IgG2a.1) endotoxin level <0,10 EU/mg, 0.5 mg/mouse Anti-TNP (mouse IgG2a.1) antibody, endotoxin level 0,001 EU/mg, 0.5 mg/mouse Etanercept® 10 mg/kg
Anti-TNP (hzIgG1) antibody, endotoxin level <0,10EU/mg, 10 mg/kg

**Serum samples**

Mice were anaesthetized by isoflurane/$O_2$/$N_2O$ and eye-bleed (app. 150 $\mu$l, max. 7.5% of estimated total blood volume) when treatment began and at termination. The blood was sampled into a 0.5 ml serum tube with clot activator (BD Microtainer) and were kept at room temperature until centrifugation (within 20 min.) at 15 000 G for 2 min. at 4°C. Serum was transferred to 1.4 ml micronic tubes and was stored at -80°C until further analysis for matrix metalloproteinase-3 (MMP3) levels using a standard ELISA assay (R&D Systems). Serum samples were analysed using the assay procedure described by the matrix metalloprotinase-3 (MMP-3) ELISA manufacture R&D systems. Serum samples required a 1:20 dilution with Calibrator diluents. Additional inflammation markers were evaluated using a 58-biomarker multi-analyte profile (RodentMAP®, Myriad, RBM, Austin, USA).

**Preparation of paws for histology**

At sacrifice paws where cut off and split sagitally between third and fourth toe to the heel bones in order to optimise fixation and decalcification. The paws were fixed in 4% paraformaldehyde for 48 hours at room temperature and placed in 70% ethanol. The paws were decalcified for 7 days in Immunocal and processed for paraffin embedding on an ASP300 tissue processor, and embedded with the cut surface down. Paraffin sections were cut at 3 µm and stained with a standard HE staining for histopathological evaluation.

**Histopathological scoring system**

Arthritic changes in the distal interphalangeal (DIP), proximal interphalangeal (PIP), metatarsal, and tarsal joints of the hind paws were assessed using a scoring system (modification of Sumariwalla et al, 2004) in which 0= normal joint architecture, 1= mild changes, synovitis and pannus front with few discrete cartilage focal erosions, 2= moderate changes, accompanying chondrocyte denucleation, loss of large areas of cartilage, eroding pannus front with some bone erosions, and synovial hyperplasia with infiltrating mononuclear cells and polymorphonuclear cells, and 3= severe bone erosions and destruction of joint architecture. The histology score index was calculated as the total additive score per mouse divided by the total number of joint areas scored per mouse (ranging from 6 to 8 joint areas per mouse).

For more specific scores of destructive changes in the paws, bone erosion and cartilage degradation were scored separately on the HE stained sections of the worst affected of the hind paws. Worst affected was defined as the paw with the highest histology score index (total additive score per paw divided by the total number of joint areas scored). If the two paws had the same histology score, the right paw was chosen. Severity of bone erosion and cartilage degradation was scored from 0-3, and the average was taken (additive score divided by the number of joint areas scored).

**Statistics**

Statistical significant effects of test compounds on clinical scoring were calculated by comparing test compound groups with control groups using the two-tailed Mann-Whitney test. Calculations were done on individual mice evaluating the area under the curve (AUC) over the total experimental time for the clinical scores using GraphPad Prism software. Mice that were sacrificed before end of experiment (after 6 treatment doses) were included in the calculation with the last observed score.

Histology data were analysed using GraphPad Prism 5, and evaluation of statistical significance of the differences in the histology score index of the treatment groups was performed using unpaired Student's t-test with Welch's correction.

**Results**

Therapeutic anti-C5aR treatment was able to halt disease development in the CIA model. A significant ($p < 0.0001$) difference in the clinical scores were observed when comparing anti-C5aR treatment with isotype control treatment. In addition, a significantly ($p = 0.0002$) reduced level of the systemic bone destruction marker MMP-3 confirmed the clinical treatment effect. Comparable inhibition of clinical scores and MMP3 level were found after etanercept treatment.

Histopathological end-point assessment of arthritic changes was performed on both hind paws from each mouse. A histology score index was calculated for each mouse. Treatment with anti-C5aR (n=18) significantly reduced the histology score index compared with the isotype control treatment (n=15) (Figure 3). There was a significant correlation between the histology score index (the treatment groups were pooled) and the clinical score at the time of sacrifice ($p < 0.0001$).

Bone erosion and cartilage degradation was evaluated on HE stained sections of worst affected hind paw from each mouse (anti-C5aR n=18, anti-TNP n=15). As seen in figure 4, treatment with anti-C5aR significantly reduces both bone erosion and cartilage degradation compared with mice treated with anti-TNP control antibody (IgG2a.1).

**Example 4**

**Effect of anti-C5aR treatment in a single paw arthritis model in mice**

**Induction of DTH-Arthritis in C57BL/6 mice**

DTH-arthritis was induced by eliciting a classical DTH reaction in one paw with methylated bovine serum albumin (mBSA), with the modification that a cocktail of type II collagen monoclonal antibodies was administered between the immunisation and challenge steps (Atkinson SA et al. (2012) Arthritis Res Ther 14(3):R134). Briefly, female C57BL/6 mice (8-10 wk old) were immunised on day -7 with mBSA (Sigma, St. Louis, MO) emulsified in complete Freund's adjuvant (CFA) (Difco, Detroit, MI) intradermally at the base of the tail. Four days later they received 1000 $\mu$g (approx.. 50 mg/kg) type II collagen (CII) mouse antibody 5-clone cocktail (Chondrex, Redmond, WA) containing the clones A2-10 (IgG2a), F10-21 (IgG2a), D8-6 (IgG2a), D1-2G (IgG2b), and D2-112 (IgG2b) intravenously in 200 $\mu$l phosphate-buffered saline (PBS). On day 0 the mice were challenged with 200 $\mu$g mBSA subcutaneously in 20 $\mu$l PBS in the right footpad. The left footpad was given 20 $\mu$l PBS only and served as control. After the footpad challenge mice develop a single paw arthritis in the right mBSA challenged footpad.

**Treatment with anti-C5aR antibody**

To investigate the effect of anti-C5aR treatment on the arthritis score, mice (n=10/group) were treated with either blocking anti-C5aR antibody (m20/70 mIgG2a.1 including 6 Fc mutations as described above) or isotype control antibody (anti-TNP - IgG2a.1), 500 $\mu$g i.p. twice weekly, 4 doses total from the day of arthritis induction (i.e. day 0).

**Quantative measurement of arthritis**

As primary efficacy read-out the Area Under Curve (AUC) of $\Delta$ paw swelling was calculated from day 0-11 post arthritis induction, where the change ($\Delta$) in paw swelling was calculated as: Right paw thickness post arthritis induction minus right paw thickness pre arthritis induction.

**Preparation of paws for histology**

At sacrifice on day 11 post arthritis induction hind paws where cut off and split sagitally between third and fourth toe to the heel bones in order to optimise fixation and decalcification. The paws were fixed in 4% paraformaldehyde for 48 hours at room temperature and placed in 70% ethanol. The paws were decalcified for 7 days in Immunocal and processed for paraffin embedding on an ASP300 tissue processor, and embedded with the cut surface down. Paraffin sections were cut at 3 $\mu$m

**Assessment of histopathological changes**

Histopathological changes in the paws were assessed on three sections from each paw stained with a conventional haematoxylin and eosin (HE) overview staining, osteoclast enzyme tartrate-resistant acid phosphatase (TRAP) staining for evaluation of bone erosion and Safranin O for demonstration of cartilage proteoglycan loss, respectively. Extra-articular infiltration of inflammatory cells (assessed on a 0-3 scale) and arthritic changes were assessed separately. Arthritic changes were assessed on metatarsal and tarsal joints, where synovitis (infiltration of inflammatory cells and hyperplasia of synovial membrane), cartilage degradation and bone erosion were scored separately on a 0-3 scale. For each of the three parameters of arthritic changes, an average between the two joint areas was calculated. Histological evaluation was performed on n=10 anti-C5aR treated mice and n=10 control antibody treated mice.

**Statistics**

For statistical analysis of the histological scores an unpaired Student's t-test with Welch's correction was used. Differences between groups were considered significant when $p \leq 0.05$ and levels of significance were assigned as *: $p \leq 0.05$, **: $p \leq 0.01$ and ***: $p \leq 0.001$.

**Results**

Anti-C5aR antibody treatment from the day of arthritis induction was shown to significantly reduce paw swelling compared to isotype control antibody (P<0.01, unpaired two-sided student's t-test, data not shown). The histopathological evaluation showed a highly significant effect of anti-C5aR treatment on arthritic changes (synovitis, cartilage degradation and bone erosion) and on extra-articular infiltration (Fig 5). Together these findings show that antiC5aR treatment can prevent arthritic changes in joints, which demonstrates the suitability of anti-C5aR as a therapeutic for the treatment rheumatoid arthritis (RA) and in addition for treatment of bone damage including bone erosion and loss of bone density.

While certain features of the invention have been illustrated and described herein, many modifications, substitutions, changes, and equivalents will now occur to those of ordinary skill in the art. It is, therefore, to be understood that the appended claims are intended to cover all such modifications and changes that fall within the true spirit of the invention.

**Example 5**

**Single dose treatment of anti-C5aR in the collagen induced arthritis (CIA) model**

To study potential rapid onset effects of anti-C5aR treatment a single dose therapeutic treatment was evaluated. Mice were treated and analysed as described above (example 3). In short mice (n=22-26/group A-C) or (n=15-16/group D) were immunized for induction of arthritis at day 0 and boosted at day 21. Single dose treatment (0.5 mg/mouse) with anti-C5aR or anti-TNP (IgG2a.1) were initiated individually when mice reached a clinical score of 2-7 corresponding to score day 1. Mice were terminated 48 hours after the treatment. Inflammation markers were evaluated in paw homogenate using a 58-biomarker multi-analyte profile (RodentMAP®, Myriad, RBM, Austin, USA). The paw homogenate was prepared by cutting off the hind paws from all mice right below the fur line on the leg. The paws were then kept cold at all times and homogenised in a buffer containing: 1 tablet Complete (Roche), 5 $\mu$l Triton X-100 (Sigma), and 500 $\mu$l FUT-175 (Calbiochem) in 0.9 % Saline (up to 50 ml). The homogenate was centrifuged two times for 15 min. at 10,000G at 4°C and the supernatant was stored at -80°C until analysis

**Results**

A single dose treatment with anti-C5aR demonstrated a halt in disease development already after 24 hours which was even more pronounced after 48 hours (figure 6A). The AUC of the clinical score was significantly lower (p=0.0003) in mice given single dose antiC5aR treatment (figure 6B).

In a further study, the histopathology and infiltration of neutrophils and macrophages was also evaluated at 48 hours after single dose treatment. As above a significant reduction in the histology score index (p=0.0006) was observed (Figure 6C). In addition the infiltration of neutrophils and macrophages were found to be significantly reduced at 48 hours (not shown). Together these data demonstrated a marked impact of single dose treatment with rapid onset effect on histopathology scores.

**Single dose treatment with anti-C5aR results in rapid changes of inflammatory biomarkers**

A panel of soluble analytes relevant to inflammation was investigated in paw homogenates and serum samples obtained from single dose treated mice 48 hours after administration of anti-C5aR. Many inflammation markers were found to be significantly reduced in mice that received anti-C5aR treatment compared to isotype (anti-TNP - IgG2a.1) treated mice (table 1).

In addition to a local response in the paw several inflammatory analytes were shown to be significantly decreased in the periphery after anti-C5aR treatment (table 1).

The matrix metalloprotinases MMP-3 and MMP-9 were both significantly (p=0.0028 and p=0.0006, respectively) reduced in the periphery corresponding to 35-45% reduction. In addition, a significant (p=0.0015) reduction of TIMP-1 corresponding to 47% reduction was found (table 1). This suggests that rapid onset effect of single dose anti-C5aR treatment on tissue remodeling and bone destruction can be detected in the periphery. As found in the paw homogenate, the chemoattractants KC (CXCL1), MCP-1 (CCL2), and MCP-5 (CCL12) were also significantly (p=0.0078, p=0.0028, p=0.021, respectively) reduced (20-50% inhibition) after single dose anti-C5aR treatment (table1).

Table 1.

| Analyte | | % reduction of mean* | |
|---|---|---|---|
| | | Paw homogenate | Serum |
| Neutrophil marker | MPO | 72 (p=0.0089) | 6 (ns) |

(continued)

| Analyte | | % reduction of mean* | |
|---|---|---|---|
| | | Paw homogenate | Serum |
| Cytokines | TNF-α | 21 (p=0.04) | LLOQ |
| | IL-6 | 69 (p=0.013) | 40 (ns) |
| | IL-17A | 55 (p=0.0061) | LLOQ |
| | IL-12p70 | 28 (p=0.0036) | LLOQ |
| | IL-3 | 52 (p=0.0094) | LLOQ |
| | IL-11 | 51 (p=0.01) | LLOQ |
| Chemoattractants | KC (CXCL1) | 69 (p=0.0037) | 46 (p=0.0078) |
| | MCP-1 (CCL2) | 63 (p=0.021) | 24 (p=0.0028) |
| | MCP-3 (CCL7) | 60 (p=0.023) | 19 (ns) |
| | MCP-5 (CCL12) | 63 (p=0.0073) | 21 (p=0.021) |
| | MIP-1b (CCL4) | 63 (p=0.02) | LLOQ |
| | M-CSF-1 | 30 (p=0.0073) | 8 (ns) |
| | IP-10 (CXCL-10) | 25 (p=0.01) | 19 (ns) |
| | GCP-2 | LLOQ | 42 (p=0.05) |
| Tissue remodelling | MMP-3# | Not analyzed | 35 (p=0.0028) |
| | MMP-9 | LLOQ | 38 (p=0.0006) |
| | TIMP-1 | 63 (p=0.019) | 47 (p=0.0015) |
| | LIF | 64 (ns) | LLOQ |
| T cell activation | CD40L | 10 (ns) | 30 (p=0.0057) |
| Cell death | APO-1/CD95 | 64 (p=0.03) | 6 (ns) |
| Acute phase reactant | CRP | 62 (p=0.0024) | 18 (ns) |

Table 1. Changes in inflammation markers after single dose anti-C5aR treatment.

Only analytes resulting in significant changes in either paw homogenate or serum are shown. *anti-C5aR treated group compared with anti-TNP treated group (t-test) 48 hours after single dose treatment. #analysed by ELISA. LLOQ; lower limit of quantification, ns; non-significant, M-CSF-1; macrophage colony-stimulating factor, GCP-2; granulocyte chemotactic protein-2; CRP; C-reactive protein, LIF; leukemia inhibitory factor.

## Example 6

### Single dose treatment of anti-C5aR in a delayed type hyper-sensitivity arthritis (DTHA) model

To further study the potential rapid onset of C5aR antagonists the effect was further evaluated in the DTHA model describe above (example 4) in a single dosage study.

Single dose treatment (0.5 mg/mouse) with anti-mC5aR or anti-TNP (mIgG2a.1) were administered i.p. The experiment was terminated 60 hours after treatment onset.

### Results

A single dose of anti-C5aR at time of arthritis induction led to a reduced paw and ankle swelling when measured 60 hrs later (figure 7A). Histopathological evaluation revealed that synovitis, cartilage destruction and bone erosion were attenuated, but extra-articular infiltration of inflammatory cells was not affected (figure 7B). The overall histopathology score was also reduced, as was the arthritis score, which is defined as the sum of all the individual evaluation parameters pertaining to an arthritic phenotype (Fig. 7C)

Together, these data demonstrate that a single dose of anti-C5aR had the ability to significantly reduce disease development and arthritic changes in DTHA

To further investigate the pathways affected by blockade of C5aR, and to investigate whether anti-C5aR lead to a reduction in activation of inflammatory cells, whole paw homogenates taken 60 hours post arthritis induction were analysed for protein levels of a range of cytokines and chemokines.

Briefly, paw homogenates were generated by homogenising paws in 1.25 ml of an ice cold homogenisation buffer containing 49.995 ml 0.9 % saline, 1 tablet complete EDTA-free protease inhibitor cocktail (Roche, Switzerland),

and 5 μl Triton X-100 (Sigma, St. Louis, MO). The paws were homogenised using a T25 Ultraturrax® homogeniser (IKA, Staufen, Germany) followed by centrifugation at 10,000 g for 15 minutes. The supernatants were decanted and centrifuged once more at 10,000 g for 15 minutes. The resulting supernatants were analysed. The homogenate supernatants were analysed undiluted for levels of inflammatory markers using bead-based Luminex® xMAP® technology with Milliplex kits from Millipore (Billerica, MA, USA) according to the manufacturer's instructions. For statistical analysis, any values below the detection limit were set to the detection limit for the analyte in question and any values above detection limit was set to the upper detection limit for the analyte in question. In the case of VCAM-1, FGF (basic) and lymphotactin the analysis was performed using a 58-biomarker multi-analyte profile (RodentMAP®, Myriad RBM, Austin, USA).

The inflammatory cytokine IL-6 was significantly decreased in the paws of antiC5aR-treated mice, while no change in IL-1β or IL-10 was observed. Furthermore, the chemoattractants CXCL1/KC, CXCL2/MIP-2, CXCL5/LIX and lymphotactin were significantly reduced in the paws of anti-C5aR-treated animals, as was G-CSF. Finally, vascular endothelial growth factor (VEGF), basic fibroblast growth factor (bFGF) and vascular cell adhesion molecule 1 (VCAM-1) were also significantly reduced in the paws of anti-C5aR-treated mice (table 2). In conclusion a single dose of anti-C5aR was able to mediate rapid changes in local inflammatory mediators, which are both markers of and important for the activation and infiltration of a variety inflammatory cell subsets and for endothelial activation.

Table 2.

| Analyte | Anti-C5aR-treated, pg/g tissue (95% CI) | IgG2a.1-treated, pg/g tissue (95% CI) | p-value | Summary |
|---|---|---|---|---|
| IL-1β | 950.2 (765.6-1135) | 935 (774.6-1035) | 0.89 | ns |
| IL-6 | 1097 (582.3-1612) | 3873 (2235-5511) | 0.0028 | ** |
| IL-10 | 4403 (3418-5388) | 4280 (3320-5239) | 0.84 | ns |
| IL-17 | 47.98 (30.28-65.67) | 86.12 (43.34-128.9) | 0.066 | ns |
| CXCL1/KC | 6854 (3919-9788) | 11630 (9374-13886) | 0.0092 | ** |
| CXCL2/MIP-2 | 7430 (4118-10742) | 20677 (13320-28035) | 0.0016 | ** |
| CXCL5/LIX | 1134 (448.5-1819) | 3301 (2227-4376) | 0.0012 | ** |
| CXCL10/IP-10 | 4403 (3418-5388) | 4280 (3320-5239) | 0.84 | ns |
| CCL3/MIP-1a | 3312 (2560-4065) | 3361 (2522-4200) | 0.92 | ns |
| CCL5/RANTE S | 1015 (863.1-1167) | 1026 (835.1-1218) | 0.91 | ns |
| G-CSF | 1681 (808.7-2562) | 6129 (2584-9675) | 0.013 | * |
| GM-CSF | 149.3 (57.3-241.4) | 260 (174.5-345.5) | 0.062 | ns |
| M-CSF | 1258 (957.7-1558) | 1040 (474.8-1604) | 0.45 | ns |
| VCAM-1 | 439 (371-506.9) | 503.4 (484-522) | 0.027 | * |
| VEGF | 74.49 (55.89-93.09) | 115.6 (89.77-141.5) | 0.0091 | ** |
| bFGF | 1567 (1048-2086) | 2349 (1705-2993) | 0.047 | * |
| Lymphotactin | 2351 (2003-2699) | 2836 (2416-3257) | 0.030 | * |

Table 2. Protein biomarkers of inflammation in paw tissue following a single dose of antiC5aR or IgG2a.1

Whole paw homogenate supernatants were analysed for protein levels of a range of inflammatory markers using multiplex analysis. See materials and methods for details. Results are shown as mean $\pm$ SEM, and n = 10/group. Differences between groups were considered significant when $p \leq 0.05$ and levels of significance were assigned as *: $p \leq 0.05$, **: $p \leq 0.01$ and ***: $p \leq 0.001$ using Student's t-test.

Claims

1. A C5aR antagonist for use in a method of treatment or prevention of rheumatoid arthritis-associated bone damage in a subject having rheumatoid arthritis, wherein the C5aR antagonist is an antibody that binds the 2nd extracellular loop of C5aR, wherein the antibody inhibits the binding of C5a to C5aR and inhibits the C5aR-mediated biological effect of C5a.

2. The C5aR antagonist for the use according to claim 1, wherein bone damage includes one or more of: bone erosion and loss of bone density.

3. The C5aR antagonist for the use according to claim 2, wherein the treatment is for reducing the rate of bone damage.

4. The C5aR antagonist for the use according to claim 2 or claim 3, wherein the treatment is for inhibiting or halting progression of bone erosion or for increasing bone density.

5. The C5aR antagonist for the use according to any of the previous claims, wherein the subject suffers from bone erosion or decreased bone density.

6. The C5aR antagonist for the use according to any of the previous claims, wherein the subject is a mammal such as a human.

7. The C5aR antagonist for the use according to any of the previous claims, wherein the C5aR mediated biological effect of C5a is selected from

    a. C5a induced neutrophil activation,
    b. C5a induced cell migration, and
    c. C5a induced neutrophil maturation.

**Patentansprüche**

1. C5aR-Antagonist zur Verwendung in einem Verfahren zur Behandlung oder Vorbeugung von rheumatoider Arthritis-assoziierter Knochenschädigung bei einem Patienten mit rheumatoider Arthritis, wobei der C5aR-Antagonist ein Antikörper ist, der die zweite extrazelluläre Schleife von C5aR bindet, wobei der Antikörper die Bindung von C5a an C5aR inhibiert und die C5aR-vermittelte biologische Wirkung von C5a hemmt.

2. C5aR-Antagonist zur Verwendung nach Anspruch 1, wobei die Knochenschädigung eine oder mehrere von: Knochenerosion und Verlust der Knochendichte umfasst.

3. C5aR-Antagonist zur Verwendung nach Anspruch 2, wobei die Behandlung zur Verringerung der Knochenschädigungsrate dient.

4. C5aR-Antagonist zur Verwendung nach Anspruch 2 oder 3, wobei die Behandlung zur Hemmung oder zum Stoppen des Fortschreitens der Knochenerosion oder zur Erhöhung der Knochendichte dient.

5. C5aR-Antagonist zur Verwendung nach einem der vorhergehenden Ansprüche, wobei der Patient an Knochenerosion oder verringerter Knochendichte leidet.

6. C5aR-Antagonist zur Verwendung nach einem der vorhergehenden Ansprüche, wobei es sich bei dem Patienten um ein Säugetier wie z.B. einen Menschen handelt.

7. C5aR-Antagonist zur Verwendung nach einem der vorhergehenden Ansprüche, wobei die C5aR-vermittelte biologische Wirkung von C5a ausgewählt ist unter

    a. C5a induzierte Neutrophilenaktivierung,
    b. C5a induzierte Zellmigration, und
    c. C5a induzierte Neutrophilenreifung.

**Revendications**

1. Antagoniste de C5aR pour une utilisation dans un procédé de traitement ou de prévention d'une lésion osseuse associée à la polyarthrite rhumatoïde chez un sujet souffrant de polyarthrite rhumatoïde, dans lequel l'antagoniste de C5aR est un anticorps qui se lie à la seconde boucle extracellulaire de C5aR, dans lequel l'anticorps inhibe la liaison de C5a à C5aR et inhibe l'effet biologique médié par C5aR de C5a.

2. Antagoniste de C5aR pour l'utilisation selon la revendication 1, dans lequel la lésion osseuse comprend un ou plusieurs parmi : une érosion osseuse et une perte de densité osseuse.

3. Antagoniste de C5aR pour l'utilisation selon la revendication 2, dans lequel le traitement est destiné à réduire le taux de lésion osseuse.

4. Antagoniste de C5aR pour l'utilisation selon la revendication 2 ou la revendication 3, dans lequel le traitement est pour inhiber ou arrêter une progression d'érosion osseuse ou pour augmenter une densité osseuse.

5. Antagoniste de C5aR pour l'utilisation selon l'une quelconque des revendications précédentes, dans lequel le sujet souffre d'une érosion osseuse ou d'une diminution de la densité osseuse.

6. Antagoniste de C5aR pour l'utilisation selon l'une quelconque des revendications précédentes, dans lequel le sujet est un mammifère tel qu'un humain.

7. Antagoniste de C5aR pour l'utilisation selon l'une quelconque des revendications précédentes, dans lequel l'effet biologique médié par C5aR de C5a est choisi parmi

a. une activation de neutrophiles induite par C5a,
b. une migration cellulaire induite par C5a, et
c. une maturation de neutrophiles induite par C5a.

# Fig. 1

| Construct # | Extracellular Domain Structure | | 7F3 | 20/70 |
|---|---|---|---|---|
| 0 | Vector only | - | - | - |
| 1 | HHHH | | +++ | - |
| 2 | mHHH | | +++ | - |
| 3 | mmHH | | +++ | - |
| 4 | mmmH | | - | +++ |
| 5 | mmmm | | - | +++ |
| 6 | HmHH | | +++ | - |
| 7 | HHmH | | - | +++ |
| 8 | HHHm | | +++ | - |
| 9 | Hmmm | | - | +++ |
| 10 | mHmm | | - | +++ |
| 11 | mmHm | | +++ | - |

**Fig. 2**

**Fig. 3**

## Fig. 4

**Bone and cartilage, worst paw**

## Fig. 5

**Fig. 6**

## Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9500164 A
- WO 03062278 A
- WO 022390 A
- WO 2009103113 A
- WO 2012168199 A
- WO 2005040219 A
- US 20050238646 A
- US 20020161201 A
- WO 2011104381 A
- WO 2011147921 A
- US 4568649 A

**Non-patent literature cited in the description**

- **WATANABE et al.** *Journal of Immunological Methods,* 1995, vol. 185, 19-29
- **JI et al.** *Immunity,* 2002, vol. 16, 157-168
- **GRANT et al.** *J Exp Med,* 2002, vol. 196, 1161-1171
- **LEE et al.** *Nat Biotechnol.,* October 2006, vol. 24 (10), 1279-84
- **LEE et al.** *Immunology and Cell Biology,* 2008, vol. 86, 153-160
- **WHITFELD et al.** *Inflammat. Res.,* 2007, vol. 56 (3), S401
- **ANDERSSON et al.** *Arthritis Rheumatism,* 2012, vol. 64 (10), S881
- **HISHIMOTO et al.** *J Exp Med.,* 07 June 2010, vol. 207 (6), 1135-43
- **WOODRUFF et al.** *Arthritis Rheum,* 2002, vol. 46, 2476-2485
- **III et al.** *Protein Eng,* 1997, vol. 10, 949-57
- **HOLLIGER ; HUDSON.** *Nat Biotechnol,* 2005, vol. 2S, 1126-1136
- **WONG.** *IDrugs,* July 1999, vol. 2 (7), 686-93
- **FINCH.** *J Med Chem.,* 03 June 1999, vol. 42 (11), 1965-74
- **WOODRUFF TM.** *FASEB J,* 2006, vol. 20 (9), 1407-1417
- **LEE et al.** *Nat Biotech,* 2006, vol. 24 (10), 1279-1284
- **ATKINSON SA et al.** *Arthritis Res Ther,* 2012, vol. 14 (3), R134